# EUROPÄISCHE PATENTSCHRIFT

(11) **EP 2 049 549 B1**
(45) Veröffentlichungstag und Bekanntmachung des Hinweises auf die Patenterteilung: **05.11.2014**
(21) Anmeldenummer: 07786667.1
(22) Anmeldetag: 13.08.2007
(51) Int. Cl.: C07F 1/00, F28D 9/04, F28F 3/12, C07C 63/307

(54) **ADSORBENS, VERFAHREN ZU DESSEN HERSTELLUNG UND VERWENDUNG IN WÄRMESPEICHERN UND WÄRMEPUMPEN**
ADSORBENT, METHOD OF PRODUCING IT AND USE IN HEAT STORES AND HEAT PUMPS
ADSORBANT, SON PROCÉDÉ DE FABRICATION ET UTILISATION DANS DES ACCUMULATEURS DE CHALEUR ET DES POMPES À CHALEUR

(30) Priorität: 11.08.2006 DE 102006037698; 18.09.2006 DE 102006043648
(43) Veröffentlichungstag der Anmeldung: 22.04.2009
(73) Patentinhaber: Fraunhofer-Gesellschaft zur Förderung der angewandten Forschung e.V., 80686 München (DE); Albert-Ludwigs-Universität Freiburg, 79098 Freiburg (DE); Johannes Gutenberg-Universität Mainz, 55099 Mainz (DE)
(72) Erfinder: HAHN, Andreas, 55246 Mainz-Kostheim (DE); SCHMIDT, Ferdinand, 79106 Freiburg (DE); HENNINGER, Stefan, 79346 Endingen (DE)
(74) Vertreter: Pfenning, Meinig & Partner GbR
(86) Internationale Anmeldenummer: PCT/EP2007/007146
(87) Internationale Veröffentlichungsnummer: WO 2008/017514

(56) Entgegenhaltungen:
- WO-A-2007/035596
- WO-A1-2006/050898
- KRAWIEC, PIOTR ET AL: "Improved hydrogen storage in the metal-organic framework Cu3(BTC)2" ADVANCED ENGINEERING MATERIALS , 8(4), 293-296 CODEN: AENMFY; ISSN: 1438-1656, 2006, XP002458331

## Beschreibung

Die Erfindung betrifft die Verwendung eines Adsorbens aus hydrophilen und porösen metallorganischen Gerüststrukturen (engl. metal-organic framework, MOF). in Wärmespeichern oder Wärmepumpen.

Adsorptionswärmespeicher bieten die Möglichkeit einer nahezu verlustfreien Speicherung von Wärme, insbesondere im Temperaturbereich bis 250 °C, über lange Zeiträume. Ein Bedarf an solchen Langzeit-Wärmespeichern besteht insbesondere im Zusammenhang mit der solarthermischen Gebäudeheizung in Erdregionen mit starker jahreszeitlicher Schwankung der solaren Einstrahlung, d.h. in allen äquatorfernen Regionen. Hier fällt im Jahresverlauf das größte Angebot der Solarwärme aus thermischen Kollektoren in den Sommer, der Heizwärmebedarf jedoch überwiegend in den Winter. Im Sinne des Aufbaus einer nachhaltigen Energieversorgung, die verstärkt auf erneuerbare Energiequellen setzt, ist die saisonale Wärmespeicherung für die Gebäudeheizung wünschenswert und ist Voraussetzung zur Erreichung hoher solarer Deckungsanteile bei der solarthermischen Gebäudeheizung.

Die Wärmespeicherung im Temperaturbereich bis ca. 250 °C ist auch für viele andere Anwendungen ein wichtiges Thema. So besteht z.B. bei der dezentralen Stromerzeugung in Anlagen mit Kraft-Wärme-Kopplung (KWK) typischerweise das Problem unterschiedlicher zeitlicher Bedarfsprofile für Strom und Wärme. Um diese Anlagen stromgeführt betreiben zu können und die erzeugte Wärme nutzen zu können, muss diese Wärme zwischengespeichert werden, bis sie gebraucht wird. Dazu werden Wärmespeicher mit hoher Energiedichte und hoher Effizienz, d.h. geringen Wärmeverlusten, benötigt.

Adsorptionswärmespeicher haben sich trotz jahrzehntelanger Forschungsanstrengungen bisher nicht am Markt durchgesetzt. Es fehlte bisher vor allem an Adsorptionsmaterialien, die im gewünschten Temperaturbereich einen großen Beladungs- und Wärmeumsatz zeigen. Die vielfach für Wärmespeicheranwendungen untersuchten und eingesetzten Zeolithe, z.B. Zeolithe mit den Strukturtypen LTA und FAU, insbesondere die kommerziell erhältlichen Zeolithe A, X und Y, erfordern typischerweise zur Desorption eine treibende Temperaturdifferenz von mindestens 100 °C zwischen Adsorber und Kondensator, also bei einer Kondensatortemperatur von 35 °C eine Desorptionstemperatur von mindestens 135 °C. Diese Temperatur kann mit typischen Flachkollektoren nicht oder nur bei sehr geringer Kollektoreffizienz erreicht werden. Es werden daher teurere Vakuumröhrenkollektoren oder strahlungskonzentrierende Kollektoren benötigt. Unter typischen Be- und Entladebedingungen eines saisonalen solaren Speichersystems, wie z.B. beschrieben in Mittelbach et al., "Solid sorption thermal energy storage for solar heating systems" (TERRASTOCK 2000, Stuttgart, 28.8.-1.9.2000), werden mit den genannten Zeolithen Beladungsumsätze von nicht mehr als 0,18 Gramm Wasser pro Gramm Zeolith erreicht. Bezogen auf die Dichte einer Schüttung des Zeoliths sind damit Speicher-Energiedichten bis etwa 150 kWh/m3 erreichbar (A. Hauer, Dissertation, TU Berlin 2002, "Beurteilung fester Adsorbentien in offenen Sorptionssystemen für energetische Anwendungen").

Mit Silikagelen werden vergleichbare Energiedichten erreicht, hier ist das Hauptproblem der geringe nutzbare Temperaturhub bei der Entladung des Speichers.

Für die saisonale solare Wärmespeicherung wird daher nach Adsorbentien gesucht, deren Wasser-Adsorptionseigenschaften zwischen denen typischer Zeolithe und typischer Silikagele liegen. Insbesondere werden Materialien gesucht, deren Adsorptionsisobaren zu einem Wasserdampfdruck von etwa 56 hPa (entsprechend einem Wasserreservoir bei 35 °C) im Temperaturbereich von etwa 60-110 °C eine Beladungsänderung von mindestens 0,2 g/g zeigen.

Metallorganische Gerüstsubstanzen wurden im Hinblick auf einen möglichen Einsatz als Hochtemperatur-Wasserstoffspeicher oder generell zur sorptiven Gasspeicherung entwickelt (U. Müller, "Metal-organic frameworks-prospective industrial applications", J. Mater. Chem. 16(2006), S. 626-636). Aufgrund der hohen Porosität und Oberfläche eignen sie sich für vielfältige weitere Einsatzgebiete die klassischerweise durch Zeolithe abgedeckt werden, wie etwa die heterogene Katalyse oder zur Gasreinigung.

Synthesevorschriften für metallorganische Gerüstsubstanzen findet man zumeist nur für Batchverfahren im Labormaßstab (K. Schlichte, "Improved synthesis, thermal stability and catalytic properties of the metal-organic framework compound Cu3(BTC)2", Micro. Meso. Mat. 73(2004), S. 81-88). Diese sind zudem meist schlecht reproduzierbar aufgrund ungenauer Dokumentation der Ausgangssubstanzen.

Mit Ausnahme des mittlerweile im technischen Maßstab hergestellten Materials MOF-5 sind noch keine Verfahren zur Formgebung der kristallinen Endprodukte entwickelt worden. Klassische Formgebungsmethoden mit anorganischen Bindern, die beispielsweise für Zeolithe entwickelt wurden, sind für metallorganische Gerüstsubstanzen nicht anwendbar, da diese Verfahren eine Temperatur von 500 °C erfordern. Metallorganische Gerüstsubstanzen sind zumeist nur bis ca. 300 °C temperaturstabil.

Krawiec, Pjotre et al, advanced Engineering materials, 8, (4), 293-296, 2006 beschreibt metallorganische Gerüststrukturen mit verbesserter Einlagerung von Wasserstoff.

Die WO 2006/050898 A2 beschreibt metallorganische Gerüststrukturen für den Einsatz als Katalysator oder als Speichermaterial.

Ausgehend hiervon war es Aufgabe der vorliegenden Erfindung, ein Adsorbens bereitzustellen, das bezüglich der Adsorption, insbesondere von Wasserdampf oder Methanol, Eigenschaften aufweist, die es für die Verwendung in einem Adsorptionswärmespeicher oder einer Adsorptionswärmepumpe besonders prädestinieren. Insbesondere sollte ein derartiges Adsorbens im thermodynamischen Zyklus eines Adsorptionswärmespeichers einen höheren spezifischen Wärmeumsatz erreichen, als die bisher aus dem Stand der Technik bekannten Materialien.

Diese Aufgabe wird durch die Verwendung mit den Merkmalen des Anspruchs 1 gelöst. Die weiteren abhängigen Ansprüche zeigen vorteilhafte Weiterbildungen auf.

Es wird ein Verfahren zur Herstellung eines Adsorbens aus hydrophilen und porösen metallorganischen Gerüststrukturen (engl. metal-organic framework, MOF) bereitgestellt, bei dem mindestens ein Übergangsmetallsalz in mindestens einem zwei- oder mehrzähnigen organischen Liganden umgesetzt wird. Das erfindungsgemäße Verfahren zeichnet sich dadurch aus, dass die Umsetzung in einem organischen Lösungsmittel mit erhöhtem Siedepunkt von mindestens 90 °C erfolgt.

Überraschenderweise konnte so gezeigt werden, dass mit der Verfahrensführung metallorganische Gerüststrukturen bereitgestellt werden können, die sich durch eine wesentlich geringere Kristallitgröße auszeichnen, was hinsichtlich der Sorptionskinetik zu deutlichen Verbesserungen des erfindungsgemäßen Adsorbens führt.

Als Übergangsmetallsalze kommen bevorzugt Salze der Übergangsmetalle ausgewählt aus der Gruppe bestehend aus Zink, Kupfer, Kobalt, Ruthenium, Osmium, Mangan, Nickel und Seltenerden-Metallen zur Anwendung.

Die organischen Liganden sind vorzugsweise solche, die verbrückende Sauerstoff-, Stickstoff- oder Schwefelatome zur Komplexierung der Übergangsmetalle aufweisen. Vorzugsweise sind diese ausgewählt aus der Gruppe der Di- und Tricarbonsäuren, wobei insbesondere Therephthalsäure, Trimesinsäure, 1,3,5-Benzoltricarboxylat (BTC) 4,4'-Bipyridin, Biphenylbisulfonsäure, 2,6-Naphthalendicarbonsäure und Mischungen hiervon bevorzugt sind.

Das Lösungsmittel, das besonders bevorzugt einen Siedepunkt im Bereich von 90 °C bis 100 °C aufweist, ist vorzugsweise ausgewählt aus der Gruppe bestehend aus Diethylenglykol, n-Butanol, Dioxan, Dimethylformamid, Diethylformamid und Mischungen hiervon.

Die Verfahrensführung erfolgt vorzugsweise bei einer Temperatur im Bereich von 70 °C bis 200 °C. Bei der Umsetzung ist ein Druck von 1 bis 50 bar bevorzugt. Die Reaktionsdauer kann dabei im Bereich von 1 h bis 14 Tage liegen. Besonders vorteilhaft bei der erfindungsgemäßen Verfahrensführung ist es, dass auf den Einsatz eines Autoklaven verzichtet werden kann.

Es wird ebenso ein Adsorbens bereitgestellt, das nach dem zuvor beschriebenen Verfahren herstellbar ist. Das Adsorbens weist dabei vorzugsweise einen mittleren Porendurchmesser im Bereich von 4 bis 50 Å, besonders bevorzugt von 10 bis 30 Å, auf.

Das Adsorbens liegt in Form von Kristalliten vor, wobei diese einen mittlere Größe im Bereich von 50 nm bis 100 µm, insbesondere von 50 nm bis 10 µm, aufweisen.

Es kann so ein Adsorbens bereitgestellt werden, das eine spezifische Oberfläche nach BET von mindestens 50 m²/g und besonders bevorzugt von mindestens 500 m²/g aufweist.

Das Adsorbens kann dabei sowohl mit Wasser oder Methanol beladen werden, wobei der Beladungsumsatz für Wasser vorzugsweise mindestens 20 Gew.-%, und besonders bevorzugt 30 Gew.-% beträgt. Die Messung des Beladungsumsatzes bezieht sich dabei auf die üblichen Zyklenbedingungen, wie sie in Wärmespeichern oder Wärmepumpen auftreten (vgl. A. Hauer, Dissertation TU Berlin 2002, "Beurteilung fester Adsorbentien in offenen Sorptionssystemen für energetische Anwendungen"). Im vorliegenden Fall wurde der Beladungsumsatz bei folgenden Zyklenbedingungen bestimmt: Desorption bei 140 °C gegenüber Kondensator bei 35 °C, Adsorption bei 35 °C gegenüber Verdampfer bei 10 °C.

Erfindungsgemäß wird die Verwendung von porösen und hydrophilen metallorganischen Gerüststrukturen aus einem Komplex eines Übergangsmetalls ausgewählt aus der Gruppe bestehend aus Zink, Kupfer, Kobalt, Ruthenium, Osmium, Mangan, Nickel und Seltenerden-Metallen mit zwei- oder mehrzähnigen organischen Liganden bzw. Mischphasen hiervon als Adsorbens in Wärmespeicher und Wärmepumpen bereitgestellt. Diese Gerüststrukturen zeigen überraschenderweise einen im Vergleich zu Materialien aus dem Stand der Technik sehr hohen Beladungsumsatz mit Wasser von vorzugsweise mindestens 20 Gew.-%, besonders bevorzugt mindestens 30 Gew.-%.

Anhand der nachfolgenden Beispiele und Figuren soll der erfindungsgemäße Gegenstand näher beschrieben werden, ohne dieses auf die hier gezeigten speziellen Ausführungsformen einschränken zu wollen.
Fig. 1 zeigt mikroskopische Aufnahmen von nach dem Stand der Technik hergestellten Metall-organischen Gitterstrukturen.
Fig. 2 zeigt mikroskopische Aufnahmen von erfindungsgemäß hergestellten Metall-organischen Gitterstrukturen.
Fig. 3 zeigt eine mikroskopische Aufnahme einer Trägerstruktur, die mit dem erfindungsgemäßen Adsorbens und Wasserglas ausgerüstet ist.

### Beispiel 1

Zur Überprüfung der generellen Eignung dieser Materialien für den Einsatz als Wärmespeichermedien wurden exemplarisch Proben des hydrophilen Materials Cu₃(BTC)₂(H₂O)₃ x H₂O hergestellt und charakterisiert.

Das hydrophile Material Cu₃(BTC)₂(H₂O)₃ x H₂O zeigte überraschenderweise eine exzellente Eignung für den Einsatz als sorptives Wärmespeichermedium auf Wasserbasis. Basis für die Herstellung war die Synthesevorschrift von K.Schlichte et al. ,Microporous and Mesoporous Materials 73 (2004), S. 81-88 bei 120 °C im Autoklaven, die sich als sehr gut reproduzierbar erwies. Die Synthesevorschrift wurde zunächst noch weiter optimiert, im Hinblick auf eine Verdopplung der Konzentration sowie Verdoppelung der Flüssigkeitsmenge, so dass je Syntheseansatz insgesamt die vielfache Menge an Material produziert werden konnte, wodurch die Eignung des Materials für den Einsatz als Wärmespeicher erst getestet werden konnte.

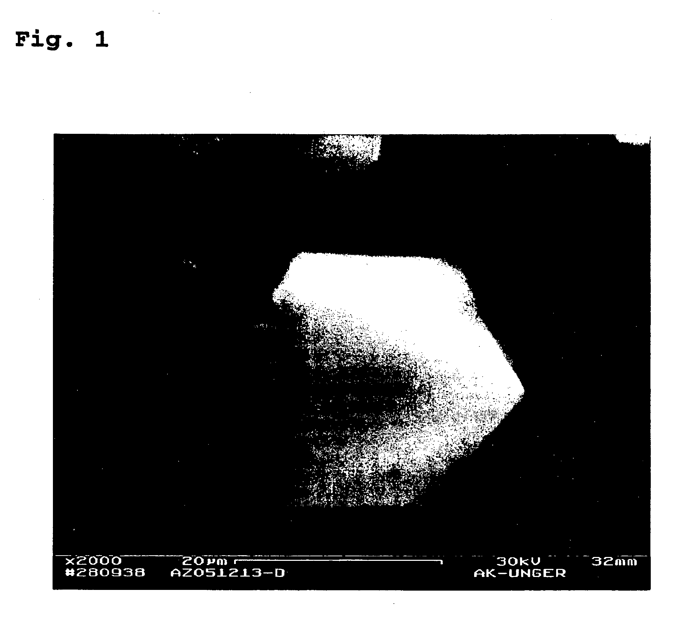

Bei der Synthese entsteht Cu₃(BTC)₂(H₂O)₃ x H₂O als ca. 20 µm große Kristalle (s. Fig. 1), die eine sehr schlechte Sorptionskinetik ergeben. Alle mittels dieser Synthese hergestellten Proben ergeben eine bereits sehr hohe Beladbarkeit mit Wasser von deutlich über 30 %, wobei das Gleichgewicht unter den Messbedingungen nicht ganz erreicht wurde, was auf eine langsame Kinetik hindeutet, für einen möglichen Einsatz in einem saisonalen Speicher aber nicht weiter hinderlich sein sollte. Im Vergleich zu Zeolithen, die dem Stand der Technik für die Wärmespeicherung entsprechen, zeigten die synthetisierten Proben eine geringere Hydrophilie, können also bei geringerer Temperatur desorbiert werden. Dadurch kann ein deutlich höherer Beladungsumsatz in einem entsprechenden Speicherzyklus erreicht werden.

### Beispiel 2

Im Hinblick auf eine bessere Kontrollierbarkeit sowie ein Upscaling ist es vorteilhaft, eine Reaktionsführung unter Normaldruck durchzuführen. Eine Reaktionsführung unter Normaldruck der Originalzusammensetzung ermöglicht allerdings nur eine Reaktionstemperatur von maximal 78 °C, die durch den Siedepunkt des Lösungsmittels Ethanol begrenzt wird. Diese Synthese ergibt nur sehr geringe Ausbeuten. Die Kristallitgröße ändert sich hierbei nicht.

Setzt man bei der Synthese nach Beispiel 1 anstatt Ethanol höhersiedende organische Lösemittel, wie etwa Diethylenglycol, n-Butanol, oder Dioxan ein, erhöht dies die mögliche Reaktionstemperatur unter Normadruck auf 100 °C, die dann durch das Wasser im Gemisch begrenzt wird. Überaschenderweise erhält man auf diese Weise sehr viel kleinere Kristallite im Bereich von 1 µm und kleiner.

Die Synthese erfolgt in folgender Weise:
In einem Kolben wird 1,75 g Kupfernitrat vorgelegt, 24 ml Wasser hinzugefügt und bis zur vollständigen Auflösung gerührt. Dazu gibt man eine Lösung von 0,84 g Trimesinsäure in 24 ml Dioxan. Anschließend wird der Kolben in ein auf 110 °C temperiertes Ölbad getaucht und mit einem Rückflusskühler versehen. Nach fünf Tagen wird die Lösung abgesaugt. Der helltürkisfarbene Niederschlag färbt sich beim Trocknen über Nacht im Vakuumtrockenschrank bei 120 °C dunkelblau. Der Strukturnachweis von Cu₃(BTC) 2xH₂O gelingt über Pulverdiffraktometrie sowie Stickstoffsorption bei 77 K.

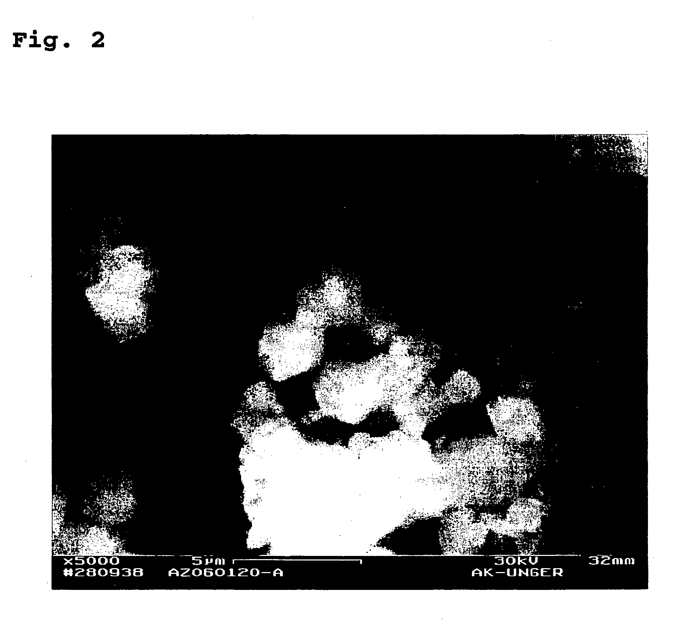

Die Untersuchung auf die Eignung als Medium im Wärmetauscher ergibt eine wesentlich bessere Kinetik sowie eine erheblich höhere Beladbarkeit bis zu 40 % (s. Fig. 2). Alternativ sind Agglomerate bzw. Verwachsungen von kleineren Kristalliten vorteilhaft für die Sorptionskinetik.

### Beispiel 3

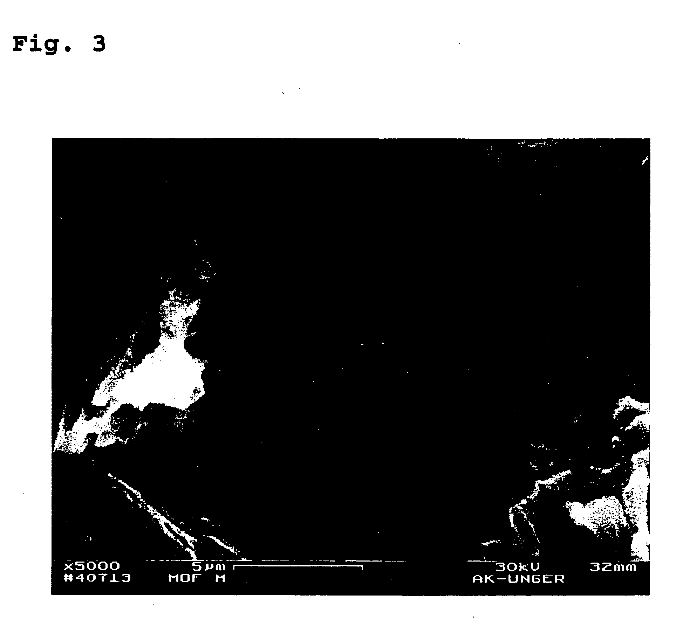

Die Kopplung des Materials an die Wärmetauscherstruktur soll über eine möglichst feste und gut wärmeleitende Anbindung erfolgen. Darüber hinaus muss das kristalline Material für das Adsorptiv gut zugänglich sein. Dies kann beispielsweise über das Aufbringen einer Mischung aus mit einem Adsorptiv beladenen Adsorbens und Na-Wasserglas geschehen, das auf das Trägermaterial aufgebracht wird. Beim anschließenden Trocknungsprozess (120 °C / Vakuum aber noch zu optimieren) härtet Natriumwasserglas in einer offenporösen Struktur aus, in der das Adsorbens eingebettet ist. Die Poren entstehen durch das Ausgasen des Adsorptivs während des Trocknungsprozesses und ermöglichen eine optimale Zugänglichkeit der Mikroporen des Adsorbens (s. Fig. 3). Weitere vorteilhafte Materialien sind organische Binder, wie beispielsweise Cellulose oder Polyvinylalkohole.

## Patentansprüche

1. Verwendung von porösen und hydrophilen metallorganischen Gerüststrukturen (MOF) aus einem Komplex eines Übergangsmetalls ausgewählt aus der Gruppe bestehend aus Zn, Cu, Co, Ru, Os, Mn, Ni und Seltenerden-Metallen mit zwei- oder mehrzähnigen organischen Liganden oder diese enthaltende Mischphasen als Adsorbens in Wärmespeichern oder Wärmepumpen.

2. Verwendung nach Anspruch 1,
**dadurch gekennzeichnet, dass** die organischen Liganden verbrückende Sauerstoff-, Stickstoff- oder Schwefel-Atome aufweisen, bevorzugt ausgewählt aus der Gruppe der Di- und Tricarbonsäuren, und besonders bevorzugt ausgewählt aus der Gruppe bestehend aus Terephthalsäure, Trimesinsäure, 1,3,5-Benzoltricarboxylat (BTC), 4,4'-Bipyridin, Biphenylbisulfonsäure, 2,6-Naphthalendicarbonsäure und Mischungen hiervon.

3. Verwendung nach einem der Ansprüche 1 oder 1,
**dadurch gekennzeichnet, dass** das Adsorbens eine spezifische Oberfläche von mindestens 50 m²/g, insbesondere von mindestens 500 m²/g aufweist.

4. Verwendung nach einem der Ansprüche 1 bis 3,
**dadurch gekennzeichnet, dass** das Adsorbens aus Kristalliten mit einer mittleren Größe im Bereich von 50 nm bis 100 µm, insbesondere 50 nm bis 10 µm, vorliegt.

5. Verwendung nach einem der Ansprüche 1 bis 4, **dadurch gekennzeichnet, dass** das Adsorbens der Adsorption von Wasser oder Methanol dient.

6. Verwendung nach dem vorhergehenden Anspruch, **dadurch gekennzeichnet, dass** das Adsorbens einen Beladungsumsatz für Wasser von mindestens 20 Gew.-%, insbesondere von mindestens 30 Gew.-% aufweist.

## Claims

1. Use of porous and hydrophilic metal-organic framework structures (MOF) made from a complex of a transition metal selected from the group consisting of Zn, Cu, Co, Ru, Os, Mn, Ni and rare earth metals having two or polydentate organic ligands or mixed phases containing these as an adsorbent in heat storage units or heat pumps.

2. Use according to claim 1,
**characterised in that** the organic ligands have bridging oxygen, nitrogen or sulphur atoms, preferably selected from the group of the dicarboxylic and tricarboxylic acids, and particularly preferably selected from the group consisting of terephthalic acid, trimesic acid, 1 3,5-benzenetricarboxylate (BTC), 4,4'-bipyridine, biphenyl disulphonic acid, 2,6-naphthalene dicarboxylic acid and mixtures thereof.

3. Use according to one of claims 1 or 2,
**characterised in that** the adsorbent has a specific surface area of at least 50m²/g, in particular of at least 500m²/g.

4. Use according to one of claims 1 to 3,
**characterised in that** the adsorbent is made out of crystallites having a median size in the range of 50nm to 100µm, in particular 50nm to 10µm.

5. Use according to one of claims 1 to 4,
**characterised in that** the adsorbent serves for the adsorption of water or methanol.

6. Use according to the preceding claim,
**characterised in that** the adsorbent has a load volume for water of at least 20% b.w., in particular of at least 30% b.w.

## Revendications

1. Utilisation de structures en charpente métallo-organique (MOF) poreuses et hydrophiles, constituées d'un complexe d'un métal de transition choisi dans le groupe consistant en Zn, Cu, Co, Ru, Os, Mn, Ni et les métaux des terres rares, et de ligands organiques bi- ou multidentés, ou de phases mixtes les contenant, en tant qu'adsorbant dans des accumulateurs de chaleur ou des pompes à chaleur.

2. Utilisation selon la revendication 1, **caractérisée en ce que** les ligands organiques comprennent des atomes d'oxygène, d'azote ou de soufre pontants, choisis de préférence dans le groupe consistant en les acides di- et tricarboxyliques, et d'une manière particulièrement préférée choisis dans le groupe consistant en l'acide téréphtalique, l'acide trimésique, le 1,3,5-benzènetricarboxylate (BTC), la 4,4'-bipyridine, l'acide biphénylebisulfonique, l'acide 2,6-naphtalène dicarboxylique et les mélanges de ceux-ci.

3. Utilisation selon l'une des revendications 1 ou 2, **caractérisée en ce que** l'adsorbant présente une aire spécifique d'au moins 50 m²/g, en particulier d'au moins 500 m²/g.

4. Utilisation selon l'une des revendications 1 à 3, **caractérisée en ce que** l'adsorbant se présente sous forme de cristallites ayant une taille de grain moyenne comprise dans la plage allant de 50 nm à 100 µm, en particulier de 50 nm à 10 µm.

5. Utilisation selon l'une des revendications 1 à 4, **caractérisée en ce que** l'adsorbant sert à l'adsorption d'eau ou de méthanol.

6. Utilisation selon la revendication précédente, **caractérisée en ce que** l'adsorbant présente un taux de charge pour l'eau d'au moins 20 % en poids, en particulier d'au moins 30 % en poids.
